# EUROPEAN PATENT SPECIFICATION

(11) **EP 1 750 648 B1**
(45) Date of publication and mention of the grant of the patent: **17.06.2009**
(21) Application number: 05729695.6
(22) Date of filing: 01.04.2005
(51) Int. Cl.: A61K 6/08

(54) **DENTAL CEMENT WITH GRAFTED POLYACID COMPOSITE PARTICLES**
DENTALZEMENT MIT AUFGEPFROPFTEN POLYSÄURE-KOMPOSITTEILCHEN
CIMENT DENTAIRE A PARTICULES COMPOSITES POLYACIDES GREFFEES

(30) Priority: 26.05.2004 EP 04012495; 01.09.2004 US 606114 P
(43) Date of publication of application: 14.02.2007
(73) Proprietor: DENTSPLY DETREY GmbH, 78467 Konstanz (DE)
(72) Inventor: KLEE, Joachim, E., 78315 Radolfzell (DE); BRUGGER, Stefan, 78315 Radolfzell (DE); WEBER, Christoph, 78464 Konstanz (DE); MÜLLER, Axel, H., E., 65193 Wiesbaden (DE); MORI, Hideharu, Yamagata 992-0057 (JP)
(74) Representative: Hartz, Nikolai
(86) International application number: PCT/EP2005/003428
(87) International publication number: WO 2005/117804

(56) References cited:
- WO-A-02/28912
- WO-A-99/01104
- WO-A-02/053107
- WO-A-02/098926
- WO-A-03/106557

## Description

### FIELD OF INVENTION

The present invention relates to dental cement compositions comprising an aqueous mixture containing composite particles with grafted polyacidic polymer chains.The present invention also relates to the use of the composite particles with grafted polyacidic polymer chains of the invention for the preparation of dental compositions curable by a glass ionomer reaction.

### BACKGROUND OF THE INVENTION

Polyalkenoate cements are known since the early nineteen seventies as powder/liquid systems consisting of poly(alkenoic acid)s and reactive ion releasing active glasses (A.D. Wilson). The most common polyacids are derived from polyacrylic acid or copolymers of acrylic and itaconic acid (S. Crisp), acrylic acid and maleic acid and to some degree a copolymer of acrylic acid with methacrylic acid (EP 0 024 056).

In the presence of water the poly(alkenoic acid) attacks the glass powder whereby metal ions such as calcium, aluminium and strontium are released under formation of intra- and intermolecular salt bridges. Generic cements have a number of important advantages for applications in dentistry such as the virtual absence of an exothermic reaction, no shrinkage during setting, no free monomer in the set composition, high dimensional stability, fluoride release and good adhesion to tooth structure.

Beside these advantageous properties the main limitation of the glass ionomer cements is their relative lack of strength and low resistance to abrasion and wear. Conventional glass ionomer cements have low flexural strength but high modulus of elasticity, and are therefore very brittle and prone to bulk fracture. Further they exhibit rather poor optical properties, In order to improve the mechanic properties especially flexural strength and fracture toughness numerous investigation were carried out in the last decades, such as the use of amino acids (Z. Ouyang, S.K. Sneckberger, E.C. Kao, B.M. Culbertson, P.W. Jagodzinski, Appl. Spectros 53 (1999) 297-301; B.M. Culbertson, D. Xie, A. Thakur, J. Macromol. Sci. Pure Appl. Chem. A 36 (1999) 681-96), application of water soluble copolymers using poly(N-vinylpyrrolidone) (D. Xie, B.M. Culbertson, G.J. Wang, J. Macromol. Sci. Pure Appl. Chem. A 35 (1998) 54761), use of poly acids with narrow molecular weight distribution (DE 100 58 829) and branched poly acids (DE 100 58 830). Further polyacids having a limited molecular mass ranging from 20,000 to 50,000 D (EP 0 797 975) and 1,000 to 50,000 D (WO 02/41845) were proposed. A further approach was the application of spherical ionomer particles (WO 00/05182).

Polycondensates or heteropolycondensates based an condensable monomer compounds of silicon were described (US 6,124,491) having a straight or branched organic chain of 4 to 50 carbon atoms and at least one double bond. Puyn et al. disclose in J. Am. Chem. Soc. 2001, 123, 9445-9446 the synthesis of block copolymers tethered to polysilesquioxane nanoparticles.

It is the problem of the present invention to provide novel dental cement systems setting by a cement reaction whereby the cured cement has improved flexural strength and fracture toughness.

### SUMMARY OF THE INVENTION

The present invention is based on the recognition that the mechanical properties of dental cements may be significantly improved by using cement compositions containing a reactive inorganic filler component such as a glass ionomer, and composite particles with grafted polyacidic polymer chains as further component of the cement reaction. Accordingly, the present invention provides a novel dental cement which sets by a cement reaction between particulate components. This setting reaction is essentially different from the conventional setting reaction between a particulate glass ionomer filler and a disperse polyacid.

Accordingly, the present invention is directed towards composite particles comprising a core (particle) and one or more grafted or tethered acid functional polymer chains. The composite particles may be formed by polymerizing specific optionally protected polymerisable acid functional monomers onto a functional particle comprising a polymerization initiation site. The polymerization process is a controlled/living polymerization process, including atom transfer radical polymerization (ATRP), reversible atom fragment transfer polymerisation (RAFT), and stable free radical polymerisation (SFRP). The composite particles can be used in dental cements as components involved in a cement reaction with a suitable reactive inorganic filler such as a reactive glass or glass ionomer.

Accordingly, the present invention provides a dental cement composition comprising
(i) a particulate reactive inorganic filler capable of leaching metal ions in the presence of an acid, and
(ii) composite particles with grafted polyacidic polymer chains, which are obtainable by a process comprising the following steps:
   (a) polymerizing one or more free radically polymerizable monomers containing optionally protected acidic groups in the presence of
      (a1) an initiatior system comprising initiator particles displaying a moiety comprising a radically transferable atom or group as a polymerization initiation site; and
      (a2) a catalyst facilitating controlled/living polymerisation, and
      (a3) optionally further polymerizable monomers,
      for forming a composite particle with grafted optionally protected polyacidic polymer chains; and
   (b) deprotecting protected acidic groups,
      for forming composite particles with grafted polyacidic polymer chains.

Further, the present invention provides a use of the composite particles with grafted polyacidic polymer chains for the preparation of dental compositions curable by a cement reaction.

### DESCRIPTION OF THE PREFERRED EMBODIMENTS

The present invention provides a dental cement composition comprising a particulate reactive inorganic filler capable of leaching metal ions in the presence of an acid. The filler is preferably a reactive glass capable of leaching metal ions and advantageously also fluoride ions. The reactive glass may be any glass conventionally used in dental cements. Preferably, a glass is used having a basic surface capable of reacting with acids in a cement reaction. Preferably, the reactive glass is a calcium or strontium fluoroalumosilicate glass. The fluoroaluminosilicate glass powder preferably has a mean particle size of 0.02 to 20 µm and is capable of reacting with particles with grafted polyacidic polymer chains. The particulate reactive inorganic filler is preferably contained in an amount of from 40 to 80 percent by weight, preferably from 50 to 70 percent by weight based on the composition.

The present invention provides a dental cement composition further comprising specific composite particles with grafted polyacidic polymer chains, which comprise a solid particle core with one or more polymer chains grafted to the particle core. The process for obtaining the composite particles with grafted polyacidic polymer chains comprises using a colloid containing specific particles as an initiator in a polymerization process. The specific particles display a moiety comprising a radically transferable atom or group capable of initiating a polymerization process, such as radical polymerization, preferably in the presence of a catalyst. The polymerization process is a controlled or living polymerization, such as atom transfer radical polymerization (ATRP), reversible atom fragment transfer polymerisation (RAFT), or stable free radical polymerizations (SFRP). The presence of functional groups comprising radically transferable atoms or groups, provides for the particles to be suitable as multifunctional particle initiators for the synthesis of composite particles by controlled/living polymerization of radically polymerizable optionally protected acid functional monomers.

A "controlled/living polymerization" is a polymerization wherein side reactions such as terminations, disproportionations and recombinations are insignificant in the polymerization process compared to chain propagation reactions. The resulting optionally protected acid functional polymer chains may be produced with molecular weight control, narrow polydispersity, end-group control and the ability to further chain extend. The term "polymer" means a homopolymer and copolymer, which may include block, random, statistical, periodic, gradient, star, graft, comb, (hyper) branched or dendritic structures. The term "polymerizable monomer" means a monomer that may be directly polymerized by the controlled/living polymerization used according to the invention and additionally a comonomer which may be copolymerized with the monomer into a copolymer.

A composite particle is a microscopic particle having a diameter in the range of from 2 nm to 20µm. An intiator particle is usually of the same size or smaller whereby the diameter is increased by the grafting of polyacidic polymer chains.

Preferably, the present invention provides composite particles with a silicon based particle core having an attached polymer comprising repeating units based on free radically polymerizable optionally protected acid functional monomers. The process of the present invention for producing such composite particles involves use of functional initiator particles comprising polymerization initiation sites. Preferably, a distribution of particles wherein 70% of the particle are within 10% of the mean particle size distribution is employed in case of a nanocondensate obtained from a silane precursor.

In the process of the invention one or more free radically polymerizable monomers containing optionally protected acidic groups are polymerized. Suitable monomers for the polymerisation process of the invention contain acidic groups optionally in protected form, and a polymerisable double bond. The acidic groups are selected from carboxylic acid groups, sulfonic acid groups, sulfuric acid groups, phosphonic acid groups, and phosphoric acid groups. Preferably, the radically polymerizable monomer is a monomer of the following formula (I) 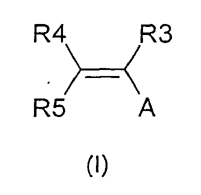 wherein A is an acidic group selected from a carboxylic acid group, a sulfonic acid group, sulphuric acid group, phosphonic acid group, and phosphoric acid group, which may optionally be protected, and which may optionally be connected to the double bond by a C₁₋₈ alkylene group; R3 is a hydrogen atom, a C₁₋₆ alkyl group or a C₃₋₆ cycloalkyl group, and R4 and R5, which may be the same or different from each other, represent a hydrogen atom, a C₁₋₆ alkyl group or a C₃₋₆ cycloalkyl group.

A is preferably a carboxyl group, a phosphoric acid group or a phosphonic acid group, which groups may be protected by a protecting group for the acidic group. R3 is preferably a hydrogen atom or a methyl group. R4 and R5 are preferably hydrogen atoms. The unsaturated carboxylic acid derivative may be an optionally protected acrylic acid or methacrylic acid such as tert.-butyl (meth)acrylic acid or n-butyl (meth)acrylic acid.

The protecting group for the acidic group A may be any suitable protecting group conventionally used for a respective acidic group. The protecting group is advantageously selected so as to be removable after the polymerisation reaction. Preferably, the liberated protecting group does not have any adverse effects on the human body. A preferred protecting group especially for a carboxyl group is a tert.-butyl group or a n-butyl group.

The radically polymerizable optionally protected acid functional monomers can be polymerized optionally in the presence of other polymerisable monomers. The polymerisation may be carried out in any sequence and into different topologies so as to generate multiple functional groups in the grafted polymer chains or to form blocks of functional monomer units. The process and product parameters discussed below for ATRP, also apply to SFRP, as well as other polymerization processes.

The free radically polymerizable monomers containing optionally protected acidic groups are polymerized in the presence of a particulate initiatior system. The polymerisation is carried out in the presence of a catalyst facilitating controlled/living polymerisation. Generic polymerisation processes are disclosed in U. S. patent applications Serial No.09/018, 554 and 09/534,827, Wang, J. S. and Matyjaszewsk, K., J. Am. Chem. Soc., vol. 117, p. 5614 (1995); Wang, J. S. and Matyjaszewsk, K., Macromolecules, vol. 28, p. 7901 (1995); K. Matyjaszewski etal., Science, vol. 272, p. 866 (1996); K. Matyjaszewski et al.,"Zerovalent Metals in Controlled/"living"Radical Polymerization,"Macromolecules, vol. 30, pp. 7348-7350 (1997); J. Xia and K. Matyjaszewski,"Controlledf"Living"Radical Polymerization. Homogenous Reverse Atom Transfer Radical Polymerization Using AIBN as the Initiator,"Macromolecules, vol. 30, pp. 7692-7696 (1997); U. S. Patent Application 09/126,768; U. S. Patents Nos. 5,807,937,5,789,487,5,910,549, 5,763,548, and 5,789,489.

A known controlled or living polymerisation process is atom transfer radical polymerization (ATRP). The ATRP polymerization of free radically polymerizable monomers for obtaining composite particles requires four components: (1) an initiator species; (2) a transition metal compound having (3) an added or associated counterion and the transition metal compound complexed with (4) ligand. According to the present invention, the initiator species is an initiator particle displaying a moiety comprising a radically transferable atom or group as a polymerization initiation site. The initiator particles may comprise aerosil particles, glass particles and nanocondensates. The initiator particle may also comprise functional silica particles and silicate based particles (e.g. obtainable according to US 6,124,491, US Application 09/359,359 and 09/534,827) further possessing initiating groups for ATRP. Preparation of such particles and the use of such nanoparticles as multi-functional initiators for polymerization process to produce particles with grafted polymers is known from WO 02/28912. In a preferred embodiment, each particle displays at least three moieties comprising a radically transferable atom or group as a polymerisation initiation site for controlled/living polymerisation. Preferably, the core of the particles comprises atoms selected from the group of silicon, titanium, zirconium, cerium, ytterbium, aluminum, tin, and yttrium. The nanoparticles have preferably a narrow particle size distribution which is narrower than the natural particle size distribution obtainable by a milling process. In an alternative embodiment, the particle size distribution corresponds to the natural particle size distribution obtainable by a conventional milling operation. The number of functional groups incorporated on the particle may be controlled by the mole ratio of initiator functional silane to non-functional silane used in the process as well as by other conventional methods. Alternatively, the amount of functional groups may be controlled by sequential reaction of the functional silane and a nonfunctional silane. A process for the incorporation of benzyl halide groups is known from U. S. Application 09/534,827. If desired, functional particles containing an attached halide group can be converted to an initiating group for SFRP by use of procedures described in commonly assigned U. S. Patent 5,910,549, or by the improved process disclosed in application 09/359,591. Substantially uniform particles with diameters between 5 to 1000 nm and 1000 initiation sites on the surface may be prepared. The number of initiating sites can be varied by varying the ratio of the surface treating agents and could vary from an average of one up to 1,000,000 or more depending on particle size and initiation site density; exemplary particles with 300 to 3000 initiating sites are preferred. It is expected that the preferred number of functional groups on each particle would be in the range of 100 to 100,000, and more preferably in the range of 300 to 30,000 to produce the advantageous properties of the composite particles. Control over the number of initiating sites on a particle allows to control the graft density of the attached polymer chains and thereby the density of the polymer chains. A high density of initiating sites provides for maximum incorporation of grafted polymer chains.

In case the particles are nanocondensates, the initiator particles are obtainable by condensing a mixture containing one or more compounds of the following formula (II) 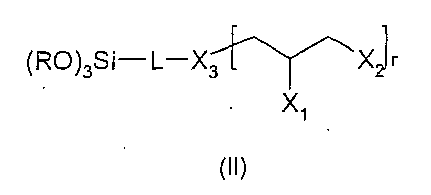 wherein the Rs, which may be the same or different, represent hydrolysable alkyl or aryl groups, L is a linker, r is 1 or 2, X1 and X2 which may be the same or different, are selected from the group of a hydroxyl goup, a halogen atom, an amino group and a thiol group, X3 is an oxygen atom, a sulfur atom or a NR" group (R" is a hydrogen atom or a C₁₋₆alkyl group) when r is 1, and X3 is a nitrogen atom when r is 2. The linker is preferably a saturated C₁₋₈ hydrocarbon chain which may contain 1 to 3 hetero atoms selected from oxygen atoms, sulfur atoms and nitrogen atoms.

A compound of formula (II) may be obtained by an addition reaction of the corresponding silane of the following formula (III)

(RO)₃Si―L―X₃(H)r (III)

wherein the Rs, X3, r and L are as defined for the corresponding formula (II), and one or more compounds of the following formula (IV) 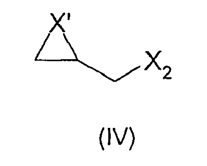 wherein X¹ is a heteroatom contained in X1, and X2 is as defiined for formula (II).

A compound of formula (III) may be used as such to prepare nanocondensates as starting material for the initiator particles of the invention.

Moreover, in case the particles are nanocondensates, the initiator particles are obtainable by condensing a mixture containing one or more compounds of the following formula (V): 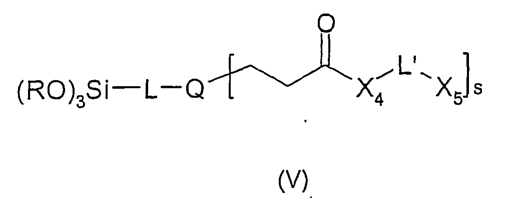 wherein the Rs, which may be the same or different, represent hydrolysable alkyl or aryl groups, L and L' which may be the same or different, are linkers, s is 1 or 2, Q is an oxygen atom, a sulfur atom or a NR" group (R" is a hydrogen atom or a C₁₋₆ alkyl group) when s is 1 and Q is a nitrogen atom when s is 2, X4 is selected from the group of O and NH, and X5 is selected from the group of a hydroxyl goup, an amino group and a thiol group, or a halogen atom. The linkers are preferably a saturated C₁₋₈ hydrocarbon chain which may contain 1 to 3 hetero atoms selected from oxygen atoms, sulfur atoms and nitrogen atoms.

A compound of formula (V) may be obtained by a Michael addition reaction of the corresponding silane of the following formula (VI)

(RO)₃Si―L―Q' (VI)

wherein the Rs and L are as defined for formula (II) and Q' is QHₛ wherein Q and s are as defined for formula (V) and one or more compounds of the following formula (VII): 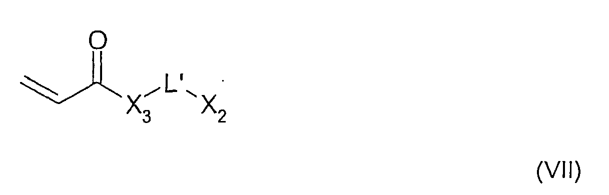 wherein X₃ and X₂ are as defined for formula (V).

A compound of formula (VI) may be used as such to prepare nanocondensates as starting material for the initiator particles of the invention.

The condensation of the silane may be carried out by acid catalysis. Suitable acids may be selected from mineral acids such as hydrofluoric acid, hydrochloric acid, phosphoric acid, and sulfuric acid. Condensation may be carried out in the presence of further hydrolysable metal compounds such as metal alkoxides selected from alkoxides of titanium, zirconium, cerium, ytterbium, aluminum, tin, and yttrium. In the absence of co-condensable metal compounds, the particle size distribution is usually narrower than in case of the presence of co-condensable metal compounds.

The initiator particle comprises a particle and a group comprising a radically transferable atom or group. Usually, a radically transferable atom may be a halogen atom. The halogen atom may be introduced *in situ.* The halogen atom may also be introduced by linking a halogen containing compound to the initiator particle. An example for a halogen containing compound is alpha-bromo-isobutyric acid which may be linked to an initiator particle by condensation to a hydroxyl group, thiol group or amine group.

The process may be catalyzed by a transition metal complex which participates in a reversible redox cycle with the initiator particle comprising the group having a radically transferable atom or group, to form a composite particle with a grafted polymer chain.

ATRP is considered to involve polymerization essentially by cleavage of the radically transferable atom or group from the initiator nanoparticle or, during the polymerization process the dormant polymer chain end, by a reversible redox reaction with a catalyst, without any strong carbon-transition (C-Cat) bond formation between the active growing polymer chain end and the transition metal complex. Within this theory as the catalyst activates the initiator or dormant polymer chain end by homolytically removing the radically transferable atom or group from the initiating nanoparticle, or growing polymer chain end, in a reversible redox reaction, an active species is formed that allows other chemistry, essentially free radical based chemistry to be conducted. The catalyst transfers a radically transferable atom or group to the active initiator molecule or growing chain end, thereby reforming a lower oxidation state catalyst complex. When free radical based chemistry occurs, a new molecule comprising a radically transferable atom or group is also formed. The counterion (s) may be the same as the radically transferable atom or group present on the initiator, for example a halide such as chlorine or bromine, or may be different radically transferable atoms or groups. An example of the latter counterion is a chloride counterion on the transition metal compound when the initiator first contains a bromine. Such a combination allows for efficient initiation of the polymerization followed by a controlled rate of polymerization, and has additionally been shown to be useful in certain crossover reactions, from one set of (co) monomers to a second set of (co) monomers, allowing efficient formation of block copolymers.

By using the process, a composite particle with grafted polymer chains is obtained. The grafted polymer chain contains acidic groups and/or protected acidic groups. In case the grafted polymer chain contains protected groups, it is preferred to deprotect protected acidic groups, for forming composite particles with grafted polyacidic polymer chains.

Since the preferred polymerization processes used in the preparation of these composite particles are controlled polymerization processes using a reactive end group to control the polymerization, the reactive end group may be available for transformation into another end group after the desired polymer is formed. On the oher hand, functional end groups of the polymer chains may be subject to further functionalisation. Accordingly, the process for obtaining composite particle with grafted polymer chains may further comrise a step of
(c) polymerizing one or more second radically polymerizable comonomers on the grafted polymer chains to form an grafted copolymer chain, and/or
(d) end-capping the grafted polyacidic polymer chains grafted on the composite particles obtained in step (b).

The end-capping may be a condensation or addition reaction. The condensation reaction or addition reaction may provide polymerizable double-bonds so that the nanoparticles obtainable according to the present invention may not only be used as components in a cement reaction with a glass ionomer component, but also as polymerisable component in an additional polymerisation reaction.

The polymerization process according to the invention may further comprise a step of isolating a composite particles with grafted polyacidic polymer chains.

The composite particles with grafted polyacidic polymer chains, which are obtainable by the process according to the invention preferably have diameters between 2 nm and 20 µm, more preferably between 2 nm and 10 µm or 2 nm and 5 µm. Preferably, the grafted polyacidic polymer chains contain at least 10 carboxylic acid groups.

Preferred composite particles of the present invention may be represented by the following formula (A)

Z[(Y(X)ₒ)ₙ]ₙ (A)

wherein

Z represents a particulate organo-silicon nanocondensate, a highly dispersed particulatesilicon dioxide or a particulate glass filler
the one or more Y denote independently from each other a bond or a divalent linker;
the one or more X denote independently from each other the following moiety 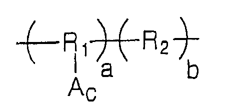 whereby
the A represent independent from each other an acidic group, selected of the group of carboxylic acids, phosphoric acid, phosphonic acid, sulfuric acid, sulfonic acid
R₁ and R₂ are substituted or unsubstituted C₁ to C₁₈ alkyl group, a substituted or unsubstituted C₃ to C₈ cycloalkyl group, a substituted or unsubstituted C4 to C18 aryl or heteroaryl group, a substituted or unsubstituted C₅ to C₁₈ alkylaryl or alkylheteroaryl group, or a substituted or unsubstituted C₇ to C₃₀ aralkyl group,
a is an integer of from 1 to 500, preferably 10 to 100
b is an integer of from 0 to 500, preferably 0 to 100;
c is an integer of from 1 to 5, preferably 1 to 2;
m is an integer of from 1 to 50, preferably 1 to 20
n is an integer of from 1 to 500, preferably 10 to 100, and
o is an integer of from 1 to 6, preferably 1 to 4.

The linker Y in formula (A) may be a substituted or unsubstituted C₁ to C₁₈ alkyl group, a substituted or unsubstituted C₃ to C₈ cycloalkyl group, a substituted or unsubstituted C4 to C18 aryl or heteroaryl group, a substituted or unsubstituted C5 to C18 alkylaryl or alkylheteroaryl group, or a substituted or unsubstituted C₇ to C₃₀ aralkyl group.

Further preferred composite particles of the present invention according to formula (A) may be represented by the following formula (B) 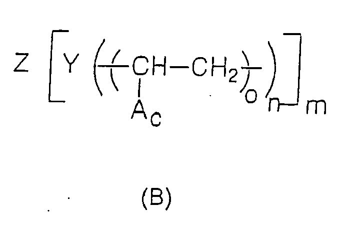 wherein
A, Y, *c*, *n, m*, *o,* and Z are as defined for formula (A).

Further preferred composite particles of the present invention according to formula (I) may be represented by the following formula (C) 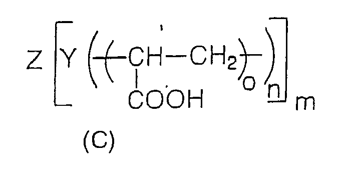 wherein Y, *c, n*, *m, o*, and Z are as defined for formula (A).

Further preferred composite particles of the present invention according to formula (A) may be represented by the following formula (D) 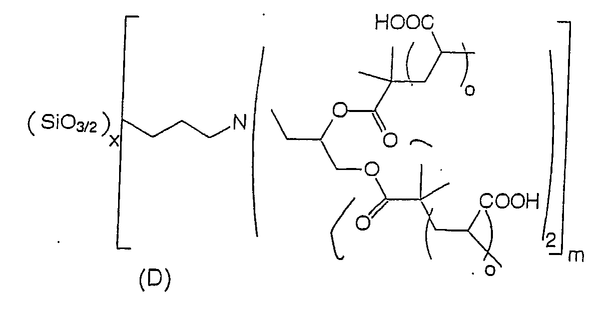 wherein
m is an integer of from 1 to 50, preferably 1 to 20
o is an integer of from 1 to 6, preferably 1 to 4
x is an integer of from 1 to 100, preferably 10 to 50.

The composite particles of the present invention are generally contained in the dental cement composition preferably in an amount of from 0.05 percent by weight to 80 percent by weight, preferably in an amount of from 0.1 percent by weight to 40 percent by weight. In a preferred embodiment of the present invention, the composite particles of the present invention are contained in the dental cement composition preferably in an amount of from 3 percent by weight to 80 percent by weight, preferably in an amount of from 10 percent by weight to 40 percent by weight. In a further preferred embodiment of the present invention, the composite particles of the present invention are contained in the dental cement composition preferably in an amount of from 0.05 percent by weight to 3 percent by weight, preferably in an amount of from 0.1 percent by weight to 1.0 percent by weight. As shown by Application Examples 2 and 3, a small amount of the composite particles of the invention may increase the mechanical properties including the flexural strength of a gass ionomer cement based on an unexpected effect.

The present invention provides a dental cement composition optionally comprising an organic or inorganic acid selected from the group of tartaric acid, maleic acid, fumaric acid, oxalic acid, phosphoric acid. The acid is used as a retardig agent for adjusting the rate of the glass ionomer reaction.

The present invention provides a dental cement composition comprising components (i) and (ii) optionally in an aqueous mixture. The ratio of the aqueous solvent containing water and optionally a further solvent to components (i) and (ii) is preferably in the range of 1:10 to 10:1 preferably 1:2 to 5:1.

The dental composition of the invention may further contain a water-soluble or water-swellable polymer or copolymer. Preferably, the water-soluble or watr-swellable polymer is selected form the group of polyacrylic acid, polyvinylalcohol, polyvinylamine, polyvinylpyrolidone. Preferably, the water-soluble copolymer is obtained by polymerization of at least two different polymerizing monomers in that manner that at least one of the polymerizing monomers contains acidic moieties selected of the group of carboxylic acids, phosphoric acid, phosphonic acid, sulfuric acid, sulfonic acid. In a preferred embodiment, the water-soluble copolymer is obtainable by polymerization of at least two different polymerizing monomers selected of the groups a) monomers such ethylene, propylene, styrene, methylmethacrylate, methylacrylate, butylmethacrylate, vinylalkylether and b) acidic monomers such as acrylic acid, methacrylic acid, vinylphosphonic acid, maleic acid, fumaric acid, maleic acid anhydride. In a further preferred embodiment of the dental composition, the water-soluble copolymer is a latex.

The dental composition of the invention may further contain additional inorganic fillers widely used for dental composite resins in combination with the reactive inorganic filler. The additional filler preferably has a mean particle size of 0.02 to 10 µm and is incapable of reacting with particles with grafted polyacidic polymer chains by a cement reaction. Examples of the additional filler are colloidal silica, quartz, feldspar, alumina, titania, borosilicate glass, kaolin, talc, calcium carbonate, calcium phosphate, and barium sulfate. Composite fillers obtained by pulverizing inorganic filler-containing polymers may be used as well. These fillers may also be used in admixture.

The dental compositions may further contain pigments. In case the dental composition is curable by a combination of a glass ionomer reaction and a polymerisation reaction, the dental composition may contain an initiator system, preferably a water-soluble initiator system. The initiator system may be a redox initiator system or a photoinitiator system.

The composition of a typical dental cement composition according to the invention is as follows:

| **Component in the dental cement** | **Percent by weight based on the total composition (preferred range)** |
|---|---|
| Particulate reactive inorganic filler | 40-80 (50-70) |
| Composite particles with grafted polyacidic polymer chains | 0.05-80 (0.1-3) or (5-20) |
| Aqueous solvent | 1-67 (5-45) |
| Additional polyacid | 0- 70 (0-50 and up to 90wt% of the composite particles used) |
| Additional filler | 0-20 (0-10) |

In case the composite particles with grafted polyacidic polymer chains of the invention contain polymerizable end-groups, the cement composition of the invention may further contain an initiator system for thermal polymerisation or photopolymerisation. Moreover, further polymerisable monomers may be incorporated into the dental cement composition of the invention in an amount of up to 20 percent by weight.

According to the present invention, the composite particles with grafted polyacidic polymer chains are used for the preparation of dental compositions curable by a cement reaction. The dental composition may be curable by a cement reaction and additionally by a further reaction. Further reactions are polymerisation reactions and polyaddition reactions. The dental composition is a multi-pack, preferably a two-pack composition. The composition may be a paste/paste system, a powder/liquid system, or a liquid/paste system. The composition is designed so as to avoid premature curing of the components. For this purpose, the reactive inorganic filler component and any acid group containing component must be formulated so as to avoid a premature cement reaction. In a first embodiment, the reactive inorganic filler is contained in a first pack and any acid group containing component is contained in a second pack. The first pack may be a powder or a paste. The second pack may be a liquid or paste. In a second embodiment, the first pack is a powder comprising the reactive inorganic filler and a solid polyacid such as polyacrylic acid, and the second pack is a paste or liquid and contains a further acid group containing component.

The present invention will now be further illustrated by the following examples.

### EXAMPLES

### Example 1

### Addition product of glycidol and 3-aminopropyl triethoxy silane (Gly-APTES)

To 149.977 g (0.6775 mol) 3-aminopropyl triethoxy silane were dropped slowly under ice cooling and stirring 100.378 g (1.3550 mol) 2,3-(epoxy)-propan-1-ol so that the temperature do not rise about 50°C. Then the mixture was were reacted for one hour at 23°C. The obtained product is soluble in solvents such as water, methanol, chloroform, DMF and THF. In the IR spectrum was observed no absorption of epoxide groups at 915 and 3050 cm⁻¹.
New absorptions were found and 3400 cm⁻¹ (OH group).
Yield: 250.355 g (100 % of th.), n₂₀^{D} = 1.4651,n_{23°C} = 1.829 ± 0.030 Pa*s
IR: 3411, 3390 (OH), 2973, 2929, 2885 (CH₂/CH₃), 1390 (CH₂/CH₃), 1078 cm⁻¹ (OH).

### Condensation to Gly-APTES-Nano

To 42.240 g (114.307 mmol) Gly-APTES adduct dissolved in 100 ml Methanol were added 6.380 g (354.449 mmol) of a 3.6 %age HF solution under stirring. The reaction mixture was stirred for additional 2 hours at ambient temperature. Then water, ethanol and methanol were removed in vacuum and the nanoparticles were dried at 40 °C at 8 mbar.
Yield: 29.531 g (100.0 % of th.)
Mₙ = 3800 g/mol The particle size of these nanoparticles is 2.8 nm.

### Reaction to nano-initiator

To an ice cooled suspension of 1.0 g Gly-APTES-Nano (0.0155 mol Hydroxyl groups) and a spatula tip of dimethylamino pyridine in 6 ml pyridine and 10 ml chloroform were added drop wise 4.1g (0.0178mol) 2-bromo isobutyrobromide during 2 h. Then the reaction mixture was stirred at room temperature for three days. The dark brown suspension was diluted with diethyl ether and extracted twice with a cold 5% aqueous NaOH. The separated organic phase was dried over Na₂SO₄. and the solvents were evaporated yielding in a brown bulk substance which was freeze-dried using dioxane resulting in 1.6g (49 %) of a yellowish powder. The nano-initiator was dissolved in 10 ml THF and purified from non-linked initiator by dialysis against THF. A further freeze-drying with dioxane yielded a brown initiator used for polymerisation.

### Polymerisation onto Gly-APTES-Nano to Nano-PAA

0.02 g Gly-APTES-Nano, 0.014 g CuBr, 2.505 g tert. butyl acrylate and 0.0169 g pentamethyl diethylene triamine (PMDETA) were polymerised in bulk at 60 C for 2.5 hours. Then the crude product was dried in vaccum, purified by dialysis with methanol and dried. Thereafter, hydrolysis of the ester moieties was made using trifluoro acetic acid.
Pₙ (arms) = 32

### Example 2 and 3

In the same manner as described in Example 1 PAA modified nanoparticles were prepared. Their properties are summarized in the following table:

| | Example 2 | Example 3 |
|---|---|---|
| Pₙ(Arms) | 65 | 98 |
| Mₙ | 147020 | 235800 |
| M_{w} | 158080 | 262030 |
| M_{z} | 171200 | 291710 |
| M_{w}/Mₙ | 1.075 | 1.111 |

### Application Example 1

A powder containing basic strontium alumo silicate glass (83 wt-%), 14.4 wt-% of Nano-PAA and 2.6 wt.-% tartaric acid was hand mixed with water in a powder liquid ratio of 5 to 1.
The glass ionomer sets within 5 minutes at 23 °C to a white solid body.

### Application Example 2

From these Nano-PAA particles experimental glass ionomer powders were prepared containing strontium alumo silicate glass (content 91.3 wt-%), polyacrylic acid (content 6.5 to 8.6 wt-%) and Nano-PAA (Pₙ (arms) = 65, content 0.1 to 2.2 wt-%).

These powders were hand mixed with a glass ionomer liquid containing water, polyacrylic acid (32 wt-%) and tartaric acid (8.5 wt-%) in a powder liquid ratio of 3.7 to 1. The resulting glass ionomer cements exhibited a working time of roughly 2.5 min and they set after roughly 2.5 minutes (measured according to ISO 9917-1).

The mechanical properties of these Nano-PAA containing cements were compared to a reference formulation containing only glass, PAA, tartaric acid and water.

| **Property** | | **Content of Nano-PAA (P, (arms) = 65)in the GIC wt-%]** | | | | | |
|---|---|---|---|---|---|---|---|
| | | **1.7** | **0.9** | **0.4** | **0.2** | **0.1** | **0.0 (reference)** |
| Working Time | [min] | ∼2.50 | ∼2.50 | ∼2.50 | nd | nd | ∼2.50 |
| Setting Time | [min] | 2.50 | 2.58 | 2.25 | 2.50 | 2.58 | 2.30 |
| Compressive Strength | [MPa] | 179 (16) | 193 (21) | 197 (7) | nd | nd | 196(19) |
| Flexural Strength | [MPa] | 35 (5) | 40 (10) | 45 (4) | 38 (3) | 42 (4) | 36 (3) |

### Application Example 3

From these Nano-PAA particles experimental glass ionomer powders were prepared containing strontium alumo silicate glass (content 91.3 wt-%), polyacrylic acid (content 6.5 to 8.6 wt-%) and Nano-PAA (Pₙ (arms) = 98, content 0.1 to 2.2 wt-%).

These powders were hand mixed with a glass ionomer liquid containing water, polyacrylic acid (32 wt-%) and tartaric acid (8.5 wt-%) in a powder liquid ratio of 3.7 to 1. The resulting glass ionomer cements exhibited a working time of roughly 2.5 min and they set after roughly 2.5 minutes (measured according to ISO 9917-1).

The mechanical properties of these Nano-PAA containing glass ionomer cements were compared to a reference formulation containing only glass, PAA, tartaric acid and water.

| **Property** | | **Content of Nano-PAA (Pₙ (arms) = 98)in the GIC [wt-%]** | | | | | |
|---|---|---|---|---|---|---|---|
| | | **1.7** | **0.9** | **0.4** | **0.2** | **0.1** | **0.0 (reference)** |
| Working Time | [min] | ~2.50 | ~2.50 | ~2.50 | nd | nd | ~2.50 |
| Setting Time | [min] | 2.50 | 2.58 | 2.50 | 2.50 | 2.67 | 2.30 |
| Compressive Strength | [MPa] | 181 (22) | 182 (18) | 183 (11) | nd | nd | 196 (19) |
| Flexural Strength | [MPa] | 41 (6) | 42 (7) | 47 (5) | 41 (6) | 41 (4) | 36 (3) |

## Claims

1. A dental cement composition comprising
(i) a particulate reactive inorganic filler capable of leaching metal ions in the presence of an acid, and
(ii) composite particles with grafted polyacidic polymer chains, which are obtainable by a process comprising the following steps:
(a) polymerizing one or more free radically polymerizable monomers containing optionally protected acidic groups in the presence of
(a1) an initiatior system comprising initiator particles displaying a moiety comprising a radically transferable atom or group as a polymerization initiation site; and
(a2) a catalyst facilitating controlled/living polymerisation, and
(a3) optionally further polymerizable monomers,
for forming a composite particle with grafted optionally protected polyacidic polymer chains; and
(b) deprotecting protected acidic groups,
for forming composite particles with grafted polyacidic polymer chains.

2. The composition of claim 1, wherein the catalyst comprises a transition metal complex.

3. The composition of claim 2, wherein the composite particles are obtainable by atom transfer radical polymerization (ATRP).

4. The composition according to claim 1, wherein the composite particles are obtainable by reversible atom fragment transfer polymerisation (RAFT) or stable free radical polymerizations (SFRP).

5. The composition according to any one of the preceding claims, wherein the acidic groups are selected from carboxylic acid groups, sulfonic acid groups, sulphuric acid groups, phosphonic acid groups, and phosphoric acid groups.

6. The composition according to any one of the preceding claims, wherein the process further comprises a step of
(c) polymerizing one or more second radically polymerizable comonomers on the grafted polymer chains to form an grafted copolymer chain, and/or
(d) end-capping the grafted polyacidic polymer chains grafted on the composite particles obtained in step (b).

7. The composition of claim 6, wherein the end-capping is a condensation or addition reaction.

8. The composition of claim 7, wherein the condensation reaction or addition reaction provides polymerizable double-bonds.

9. The composition according to any one of the preceding claims wherein the free radically polymerizable monomer containing optionally protected acidic groups is a protected unsaturated carboxylic acid derivative.

10. The composition according to any one of the preceding claims wherein the unsaturated carboxylic acid derivative is an optionally protected acrylic acid or methacrylic acid.

11. The composition according to any one of the preceding claims wherein the initiator particles are selected from aerosil particles, glass particles and nanocondensates.

12. The composition according to any one of the preceding claims wherein each initiator particle displays at least three moieties comprising a radically transferable atom or group as a polymerisation initiation site for controlled/living polymerisation.

13. The composition according to any one of the preceding claims wherein the initiator-particles are obtainable by condensing a mixture containing at least one compound of the following formulae:
(1) a compound of the following formula (II) 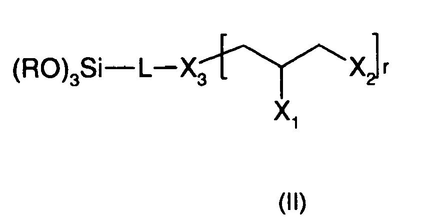 wherein the Rs, which may be the same or different, represent hydrolysable alkyl or aryl groups,
L is a linker,
r is 1 or 2,
X1 and X2 which may be the same or different, are selected from the group of a hydroxyl goup, a halogen atom, an amino group and a thiol group,
X3 is an oxygen atom, a sulfur atom or a NR" group (R" is a hydrogen atom or a C₁₋₆ alkyl group) when r is 1, and X3 is a nitrogen atom when r is 2.
(2) a compound of the following formula (III)
(RO)₃Si―L―X₃(H)r (III)
wherein the Rs, X3, r and L are as defined for formula (II);
(3) a compound of the following formula (V): 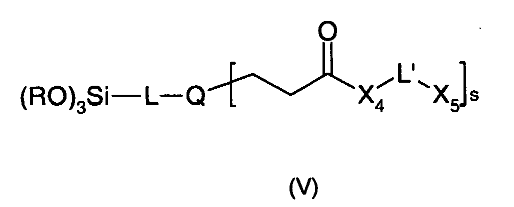 wherein the Rs, which may be the same or different, represent hydrolysable alkyl or aryl groups,
L and L' which may be the same or different, are linkers,
s is 1 or 2,
Q is an oxygen atom, a sulfur atom or a NR" group (R" is a hydrogen atom or a C₁₋₆ alkyl group) when s is 1 and Q is a nitrogen atom when s is 2,
X4 is selected from the group of O and NH, and
X5 is selected from the group of a hydroxyl goup, an amino group and a thiol group, or a halogen atom; or
(4) a compound of of the following formula (VI)
(RO)₃Si―L―Q' (VI)
wherein the Rs and L are as defined for formula (II) and Q' is QHₛ wherein Q and s are as defined for formula (V).

14. The composition according to any one of the preceding claims wherein the unsaturated carboxylic acid derivative is an optionally protected carboxylic acid selected from tert.-butyl (meth)acrylic acid and n-butyl (meth)acrylic acid.

15. The composition according to any one of the preceding claims, wherein the composite particle comprises silicon, titanium, aluminum, zirconium, vanadium, cerium, tin or yttrium.

16. The composition according to any one of the preceding claims, wherein the initiator and/or composite particles have a narrow particle size distribution.

17. The composition according to any one of the preceding claims, further comprising
(e) isolating a composite particles with grafted polyacidic polymer chains.

18. The composition according to any one of the preceding claims, wherein the particles have diameters between 2nm and 20 µm.

19. The composition according to any one of the preceding claims, wherein the particles have diameters between 2 and 200 nm.

20. The dental composition according to any one of claims 1 to 19 wherein the reactive inorganic filler is a Ca or Sr fluoroalumosilicate glass.

21. Use of the composite particles with grafted polyacidic polymer chains obtainable according to a process defined by any one of claims 1 to 20 for the preparation of dental compositions curable by a glass ionomer reaction or a combination of a glass ionomer reaction and a radical polymerisation.

22. A dental cement composition according to claim 1, which comprises
0.05 to 3 % by weight based on the total weight of the cement composition of composite particles with grafted polyacidic polymer chains.

23. The dental cement composition according to claim 22 which is further **characterized by** the features according to one or more of claims 2 to 20.

## Patentansprüche

1. Dentalzement-Zusammensetzung, umfassend
(i) einen teilchenförmigen reaktiven anorganischen Füllstoff, der in Gegenwart einer Säure Metallionen abgeben kann, und
(ii) Komposit-Teilchen mit gepropften polyaziden Polymerketten, die durch ein Verfahren, umfassend die folgenden Stufen, erhältlich sind:
(a) Polymerisation eines oder mehrerer radikalisch polymersierbarer Monomere, die gegebenenfalls geschützte Säuregruppen enthalten können, in Gegenwart von
(a1) einem Initiatorsystem, umfassend Initiatorteilchen, die eine Gruppe aufweisen, die ein radikalisch übertragbares Atom oder Gruppe als Polymerisationsinitüerungsstelle umfasst;
(a2) einem Katalysator, der eine kontrollierte/lebende Polymerisation ermöglicht, und
(a3) gegebenenfalls weiteren polymerisierbaren Monomeren, zur Bildung eines Kompositteilchens mit gepropften, gegebenenfalls geschützten polyaziden Polymerketten; und
(b) Entschützen von geschützten Säuregruppen, zur Bildung von Kompositteilchen mit gepropften polyaziden Polymerketten.

2. Die Zusammensetzung nach Anspruch 1, wobei der Katalysator einen Übergangsmetallkomplex umfasst.

3. Die Zusammensetzung nach Anspruch 2, wobei die Kompositteilchen erhältlich sind durch Atomtransfer-Radikalpolymerisation (ATRP).

4. Die Zusammensetzung nach Anspruch 1, wobei die Kompositteilchen erhältlich sind durch umgekehrte Atomfragment-Transferpolymerisation (RAFT) oder stabile Radikalpolymerisation (SFRP).

5. Die Zusammensetzung nach einem der vorstehenden Ansprüche, wobei die Säuregruppen ausgewählt sind aus Karbonsäuregruppen, Sulfonsäuregruppen, Schwefelsäuregruppen, Sulfonsäureestergruppen, Phosphonsäuregruppen und Phosphorsäuregruppen.

6. Die Zusammensetzung nach einem der vorstehenden Ansprüche, wobei das Verfahren weiterhin umfasst eine Stufe einer
(c) Polymerisation einer oder mehrerer zweiter radikalisch polysierbarer Comonomere auf die gepfropften Polymerketten, um eine gepropfte Copolymerkette zu bilden, und/oder
(d) eine Endverkappung der gepropften polyaziden Polymerketten, die auf die in Stufe (b) erhaltenen Kompositteilchen gepropft wurden.

7. Die Zusammensetzung nach Anspruch 6, wobei die Endverkappung eine Kondensations- oder Additionsreaktion ist.

8. Die Zusammensetzung aus Anspruch 7, wobei die Kondensationsreaktion oder die Additionsreaktion polymersierbare Doppelbindungen bereitstellt.

9. Die Zusammensetzung nach einem der vorstehenden Ansprüche, wobei das radikalisch polymerisierbare Monomer, das gegebenenfalls geschützte Säuregruppen enthält, ein geschütztes ungesättigtes Karbonsäurederivat ist.

10. Die Zusammensetzung nach einem der vorstehenden Ansprüche, wobei das ungesättigte Karbonsäurederivat eine gegebenenfalls geschützte Acrylsäure oder Methacrylsäure ist.

11. Die Zusammensetzung nach einem der vorstehenden Ansprüche, wobei die Initiatorteilchen ausgewählt sind aus Aerosilteilchen, Glasteilchen und Nanokondensaten.

12. Die Zusammensetzung nach einem der vorstehenden Ansprüche, wobei jedes Initiatorteilchen mindestens drei Gruppen zeigt, die ein radikalisch übertragbares Atom oder Gruppe als Polymerisationsinitiationsstelle zur kontrollierten/lebenden Polymerisation aufweist.

13. Die Zusammensetzung nach einem der vorstehenden Ansprüche, wobei die Initiatorteilchen erhältlich sind durch Kondensation eines Gemisches, das mindestens eine Verbindung der folgenden Formel enthält:
(1) Eine Verbindung der folgenden Formel (II) 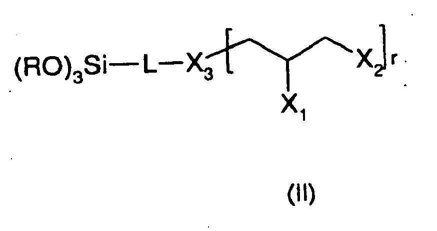 wobei
die Rs, die gleich oder verschieden sein können, hydrolisierbare
Alkyl-oder Arylgruppen darstellen,
L einen Linker bedeutet,
r 1 oder 2 bedeutet,
X1 und X2, die gleich oder verschieden sein können, ausgewählt sind aus der Gruppe bestehend aus einer Hydroxylgruppe, einem Halogenatom, eine Aminogruppe und einer Thiolgruppe,
X3 ein Sauerstoffatom, ein Schwefelatom oder eine NR" Gruppe ist (R" ist ein Wasserstoffatom oder eine C₁₋₆Alkylgruppe) wenn r 1 ist, und X3 ist ein Stickstoffatom wenn r 2 bedeutet.
(2) Eine Verbindung der folgenden Formel (III)
(RO)₃Si―L―₃(H)r (III)
wobei die Rs, X3, r und L wie in Formel (II) definiert sind;
(3) Eine Verbindung der folgenden Formel (V): 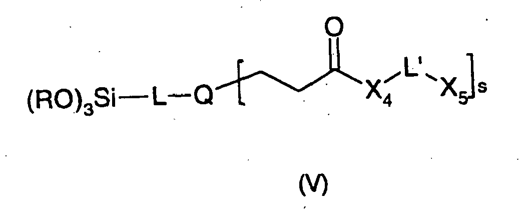 wobei die Rs, die gleich oder verschieden sein können, hydrolysierbare Alkyl oder Arylgruppen darstellen,
L und L', die gleich oder verschieden sein können, Linker bedeuten,
s 1 oder 2 bedeutet,
Q ein Sauerstoffatom, ein Schwefelatom oder eine NR" Gruppe darstellt (R" ist ein Wasserstoffatom oder eine C₁₋₆ Alkylgruppe) wenn s 1 bedeutet und Q ist ein Stickstoffatom wenn s 2 bedeutet,
X4 ist ausgewählt aus der Gruppe aus O oder NH, und
X5 wird ausgewählt aus der Gruppe bestehend aus einer
Hydroxylgruppe, einer Aminogruppe und einer Thiolgruppe, oder einem Halogenatom; oder
(4) Eine Verbindung der folgenden Formel (VI)
(RO)₃Si―L―Q' (VI)
wobei dir Rs und L wie für Formel (II) definiert sind und Q' QHₛ bedeutet, wobei Q und s wie für Formel (V) definiert sind.

14. Die Zusammensetzung nach einem der vorstehenden Ansprüche, wobei das ungesättigte Karbonsäurederivat eine gegebenenfalls geschützte Karbonsäure ist, die ausgewählt wird aus tert.-Butyl(meth)acrylsäure und n-Butyl(meth)acrylsäure.

15. Die Zusammensetzung nach einem der vorstehenden Ansprüche, wobei die Kompositteilchen Silizium, Titan, Aluminium, Zirkonium, Vanadium, Cer, Zinn oder Yttrium umfassen.

16. Die Zusammensetzung nach einem der vorstehenden Ansprüche, wobei die Initiator- und/oder Kompositteilchen eine enge Teilchengrößenverteilung aufweisen.

17. Die Zusammensetzung nach einem der vorstehenden Ansprüche, die weiterhin durch (e) Isolierung eines Kompositteilchens mit gepropften Polysäurepolymerketten erhältlich ist.

18. Die Zusammensetzung nach einem der vorstehenden Ansprüche, wobei die Teilchen ein Durchmesser zwischen 2 nm und 20 µm aufweisen.

19. Die Zusammensetzung nach einem der vorstehenden Ansprüche, wobei die Teilchen einen Durchmesser im Bereich von 2 bis 200 nm aufweisen.

20. Die Dentalzusammensetzung nach einem der Ansprüche 1 bis 19, wobei der reaktive anorganische Füllstoff ein Calcium- oder Strontiumfluoroalumosilikatglas ist.

21. Verwendung der Kompositteilchen mit gepropften polyaziden Polymerketten, die erhältlich sind nach einem Verfahren, das in einem der Ansprüche 1 bis 20 definiert wird, zur Herstellung von Dentalzusammensetzungen, die in einer Glasionomerreaktion härtbar sind oder durch eine Kombination aus einer Glasionomerreaktion und einer radikalischen Polymerisation.

22. Dentalzement-Zusammensetzung gemäß Anspruch 1, die 0,05 bis 3 Gewichtsprozent Kompositteilchen mit gepropften polyaziden Polymerketten bezogen auf das Gesamtgewicht der Zementzusammensetzung umfasst.

23. Die Dentalzementzusammensetzung gemäß Anspruch 22, die weiterhin **gekennzeichnet ist durch** die Merkmale gemäß einem oder mehreren der Ansprüche 2 bis 20.

## Revendications

1. Composition de ciment dentaire comprenant
(i) une charge minérale réactive particulaire capable de libérer des ions métalliques par lixiviation en présence d'un acide, et
(ii) des particules composites ayant des chaînes polymères polyacides greffées, qui peuvent être obtenues par un procédé comprenant les étapes suivantes :
(a) polymériser un ou plusieurs monomères polymérisables par voie radicalaire contenant des groupes acides facultativement protégés en présence de
(a1) un système initiateur comprenant des particules d'initiateur présentant un groupement comprenant un atome ou groupe transférable par voie radicalaire en tant que site d'initiation de polymérisation ; et
(a2) un catalyseur facilitant la polymérisation contrôlée/vivante, et
(a3) facultativement d'autres monomères polymérisables,
pour former une particule composite ayant des chaînes polymères polyacides greffées facultativement protégées ; et
(b) déprotéger les groupes acides protégés, pour former des particules composites ayant des chaînes polymères polyacides greffées.

2. Composition selon la revendication 1, dans laquelle le catalyseur comprend un complexe de métal de transition.

3. Composition selon la revendication 2, dans laquelle les particules composites peuvent être obtenues par polymérisation radicalaire par transfert d'atome (ATRP).

4. Composition selon la revendication 1, dans laquelle les particules composites peuvent être obtenues par polymérisation à transfert réversible par addition-fragmentation (RAFT) ou polymérisations par radicaux libres stables (SFRP).

5. Composition selon l'une quelconque des revendications précédentes, dans laquelle les groupes acides sont choisis parmi des groupes acide carboxylique, des groupes acide sulfonique, des groupes acide sulfurique, des groupes acide phosphonique et des groupes acide phosphorique.

6. Composition selon l'une quelconque des revendications précédentes, dans laquelle le procédé comprend de plus une étape de
(c) polymérisation d'un ou plusieurs seconds comonomères polymérisables par voie radicalaire sur les chaînes polymères greffées pour former une chaîne copolymère greffée, et/ou
(d) coiffage terminal des chaînes polymères polyacides greffées qui sont greffées sur les particules composites obtenues dans l'étape (b).

7. Composition selon la revendication 6, dans laquelle le coiffage terminal est une réaction de condensation ou d'addition.

8. Composition selon la revendication 7, dans laquelle la réaction de condensation ou la réaction d'addition fournit des doubles liaisons polymérisables.

9. Composition selon l'une quelconque des revendications précédentes, dans laquelle le monomère polymérisable par voie radicalaire contenant des groupes acides facultativement protégés est un dérivé d'acide carboxylique insaturé protégé.

10. Composition selon l'une quelconque des revendications précédentes, dans laquelle le dérivé d'acide carboxylique insaturé est un acide acrylique ou acide méthacrylique facultativement protégé.

11. Composition selon l'une quelconque des revendications précédentes, dans laquelle les particules d'initiateur sont choisies parmi des particules d'aérosil, des particules de verre et des nanocondensats.

12. Composition selon l'une quelconque des revendications précédentes, dans laquelle chaque particule d'initiateur présente au moins trois groupements comprenant un atome ou groupe transférable par voie radicalaire en tant que site d'initiation de polymérisation pour une polymérisation contrôlée/vivante.

13. Composition selon l'une quelconque des revendications précédentes, dans laquelle les particules d'initiateur peuvent être obtenues par condensation d'un mélange contenant au moins un composé des formules suivante :
(1) un composé ayant la formule (II) suivante 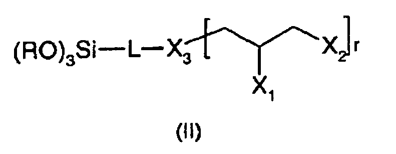 où les R, qui peuvent être identiques ou différents, représentent des groupes alkyle ou aryle hydrolysables,
L est un groupe de liaison,
r est 1 ou 2,
X₁ et X₂, qui peuvent être identiques ou différents, sont choisis dans le groupe formé par un groupe hydroxyle, un atome d'halogène, un groupe amino et un groupe thiol,
X₃ est un atome d'oxygène, un atome de soufre ou un groupe NR" (R" est un atome d'hydrogène ou un groupe alkyle en C₁ à C₆) lorsque r est 1, et X₃ est un atome d'azote lorsque r est 2.
(2) un composé ayant la formule (III) suivante
(RO)₃Si―L―X₃(H)r (III)
où les R, X₃, r et L sont tels que définis pour la formule (II) ;
(3) un composé ayant la formule (V) suivante : 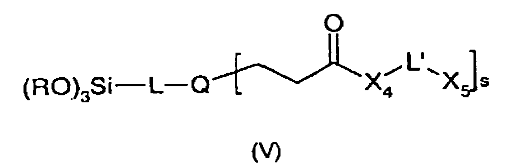 où les R, qui peuvent être identiques ou différents, représentent des groupes alkyle ou aryle hydrolysables,
L et L', qui peuvent être identiques ou différents, sont des groupes de liaison,
s est 1 ou 2,
Q est un atome d'oxygène, un atome de soufre ou un groupe NR" (R" est un atome d'hydrogène ou un groupe alkyle en C₁ à C₆) lorsque s est 1 et Q est un atome d'azote lorsque s est 2,
X₄ est choisi dans le groupe formé par O et NH, et
X₅ est choisi dans le groupe formé par un groupe hydroxyle, un groupe amino et un groupe thiol, ou un atome d'halogène ; ou
(4) un composé ayant la formule (VI) suivante
**(RO)₃Si—L—Q'** (VI)
où les R et L sont tels que définis pour la formule (II) et Q' est QHₛ où Q et s sont tels que définis pour la formule (V).

14. Composition selon l'une quelconque des revendications précédentes, dans laquelle le dérivé d'acide carboxylique insaturé est un acide carboxylique facultativement protégé choisi parmi l'acide (méth)acrylique tert-butylé et l'acide (méth)acrylique n-butylé.

15. Composition selon l'une quelconque des revendications précédentes, dans laquelle la particule composite comprend du silicium, du titane, de l'aluminium, du zirconium, du vanadium, du cérium, de l'étain ou de l'yttrium.

16. Composition selon l'une quelconque des revendications précédentes, dans laquelle les particules d'initiateur et/ou les particules composites ont une distribution granulométrique étroite.

17. Composition selon l'une quelconque des revendications précédentes, comprenant de plus
(e) l'isolement des particules composites ayant des chaînes polymères polyacides greffées.

18. Composition selon l'une quelconque des revendications précédentes, dans laquelle les particules ont des diamètres compris entre 2 nm et 20 µm.

19. Composition selon l'une quelconque des revendications précédentes, dans laquelle les particules ont des diamètres compris entre 2 et 200 nm.

20. Composition dentaire selon l'une quelconque des revendications 1 à 19, dans laquelle la charge minérale réactive est un verre de fluoroaluminosilicate de Ca ou Sr.

21. Utilisation des particules composites ayant des chaînes polymères polyacides greffées pouvant être obtenues selon un procédé défini par l'une quelconque des revendications 1 à 20 pour la préparation de compositions dentaires durcissables par une réaction de verre ionomère ou une combinaison d'une réaction de verre ionomère et d'une polymérisation radicalaire.

22. Composition de ciment dentaire selon la revendication 1, qui comprend 0,05 à 3% en poids, par rapport au poids total de la composition de ciment, de particules composites ayant des chaînes polymères polyacides greffées.

23. Composition de ciment dentaire selon la revendication 22, qui est en outre **caractérisée par** les caractéristiques selon une ou plusieurs des revendications 2 à 20.
